# EUROPEAN PATENT SPECIFICATION

(11) **EP 1 112 730 B1**
(45) Date of publication and mention of the grant of the patent: **25.04.2007**
(21) Application number: 00128718.4
(22) Date of filing: 29.12.2000
(51) Int. Cl.: A61F 13/472, A61F 13/475, A61F 13/15

(54) **A sanitary napkin**
Damenbinde
Serviette hygiénique

(30) Priority: 29.12.1999 BR 9903096
(43) Date of publication of application: 04.07.2001
(73) Proprietor: Johnson & Johnson Industria e Comercio Ltda., 05501-030 Sao Paulo, SP (BR)
(72) Inventor: Ribeiro Carvalho, Antonio Carlos, Taubaté, Sao Paulo (BR)
(74) Representative: Metten, Karl-Heinz

(56) References cited:
- EP-A- 0 426 197
- WO-A-97/05840
- WO-A-97/45082
- WO-A-98/58613
- WO-A-99/55272
- FR-A- 2 713 083

## Description

The present invention is related to a sanitary napkin, particularly a woman's sanitary napkin, and more specifically an intimate woman's sanitary napkin provided with a collecting and conveying element for the menstrual flow.

### Description of the State of the Art

As known in the art, the woman's sanitary napkins (also simply designated as napkins in the following text) are generally disposable and used to collect and retain vaginal exudates, especially menstrual blood and intermenstrual secretions.

Usually, such napkins are comprised of a substantially elongated absorbent core, sandwiched between a top layer and a lining layer.

The top layer contacts the user's pelvic area and is generally made of a pervious material that does not cause any irritation to the user's skin.

The purpose of the lining layer, opposite the top layer, is to prevent the fluid retained in the absorbent core from leaking into the user's clothes, being generally made of an impervious material.

The attachment of the sanitary napkin to the user's underwear is generally carried out through adhesive areas on the outer surface of the lining layer, which are stuck to the inner surface of the area in the user's panties between her thighs. In addition, as an aid to such attachment, the napkin may be provided with side wings provided with adhesive areas that are folded and releasably adhered to the outer surface of the area in the user's panties between her thighs.

An undesirable aspect concerning the napkins known in the present art is the occurrence of leaking when the napkin is not well fitted in the user's pelvic area. When such situation takes place, the evacuation of vaginal exudate can firstly reach areas near the edges instead of the central area, and therefore brings about a leakage because it cannot be promptly absorbed, and then leaks.

Still another cause of leakages during the use of napkins known in the state of the art is the distance between the point in the body where the body exudate leaves and the napkin surface - the larger the distance the greater the chance of its slipping through the body surface itself and leaking.

U.S. Patent No 5,702,380, that represents a prior art very close to the present invention, describes a woman's sanitary napkin provided with a longitudinal central protuberance, said protuberance being adapted to be located between the user's labia majora, but not penetrating into her vagina. The disclosure of said patent teaches that the central protuberance 14 is an element provided with several components, viz, an absorbent packing 34 which is covered on its surface by a permeable cover layer 28 and contains the central rod 56 of an inverted T shaped fluid drawing device 54 inside same.

This patent has a number of drawbacks:
- the central protuberance 14 is aligned with the rest of the napkin element, in such a way that when the napkin is moved according to the movements of the user, the protuberance is also moved and rubbed against the inner walls of the labia majora and might cause discomfort to the user;
- the central protuberance 14 is provided with absorbent material, thus, storing fluid and providing the user with a damp feeling
- the central protuberance 14 is of complex construction, either in view of its integration with the napkin surface (figures 3A, 3B and 3C) or due to the existence of the fluid drawing device 54 inside same.

Also, the American paper Statutory Invention Registration H1614 can be mentioned which discloses a woman's napkin provided with two superposed adjacent absorbent structures, the first one being protuberant and approximately tubular in the longitudinal direction, the second one being planar and elongated, wherein the first one is more comfortable and smoother than the second one, so that during the use it contacts the user's labia majora, being partially inserted between them. This achievement also provides faulty aspects, as follows:
- the first absorbent structure, although being adjustable, provides an apparently excessive volume to be comfortably inserted between the user's labia majora without being inconvenient;
- the first absorbent structure is attached over the second absorbent structure, thus causing a movement according to the user's movement, bringing about discomfort and even pain to the user during use;
- the first absorbent structure retains fluids, and therefore provides the user with a damp feeling. In WO 99/55272 also a longitudinally absorbent hump is disclosed in Figure 36 thereof, which is not joined to the basepad between its ends. However, the hump still is of excessive volume, having in particular ends of excessive thickness, thus being uncomfortably inserted between the user's labia majora. The hump is provided with absorbent material, thus storing fluid and providing the user with a damp feeling. The same disadvantages are inherent to the napkin disclosed in WO 98/58613. In particular the embodiment shown in Figure 6 thereof will cause discomfort and even pain to the user during use as a stiff insert made of a very stiff material like plastic, wood or metal is attached orthogonal to the pad by gluing or welding and thus is moved and rubbed against the inner walls of the labia majora according to the movements of the user. WO 97/45082 describes a sanitary napkin comprising a base pad with an absorbent layer and a longitudinal collecting element. The collecting element is a barrier cuff that is obtained by folding an absorbent sheet that is a part of the absorbent layer of the base pad. It is firmly connected to the base pad and is supposed to prevent the sanitary napkin from widthwise slippage. FR 2 713 083 describes a sanitary napkin with conveying elements consisting of fiber bundles of a suitable material, which is preferably non-absorptive. The fibers are anchored within the napkin. EP 0 594 059 describes a sanitary napkin comprising a nonabsorbent sheath, which is supposed to be at least partially inserted into the vagina of the user. In a preferred embodiment the sheath is an integral part of the layer that forms the garment facing surface (a so called *"baffle")* of the sanitary napkin.

### Brief Description and Objects of the Invention

In view of the problems found in the state of the art, an object of the present invention is to provide a sanitary napkin provided with an elongated collecting and conveying element for the menstrual flow which is substantially disposed along the longitudinal axis of the sanitary napkin.

Another object of the present invention is a sanitary napkin cooperating with the user's vulva area in order to collect and convey the flow of vaginal exudates to the central area of the absorbent pad surface, thus minimizing the possibilities of leakage due to the early displacement of the exudates towards the sides of the article.

Still another object of the present invention is a sanitary napkin provided with a collecting and conveying element for the menstrual flow that during the use penetrates at less partially between the user's labia majora in a less aggressive way and almost not subject to the movement of the user's body.

Such objectives are reached by a sanitary napkin characterized by the features of independent claim 1. Preferred embodiments are continued in sub-claims 2 to 11.

Concisely, some aspects of the invention are the collection of the vaginal exudate, the conveyance of the exudate to the absorbent medium but not the alignment of the collecting and conveying element with the absorbent element, and the cooperation of the sanitary napkin with the user's vulva area.

Within a particular embodiment of the present invention, the laminar collecting and conveying element for vaginal exudates (blood, urine, intermenstrual secretions, and the like.) is embodied as a non-woven veil preferably joined only to the ends of the sanitary napkin by attachment points substantially disposed along the central longitudinal axis of the sanitary napkin. When put in use, the sanitary napkin assumes longitudinally arcuated configuration following the anatomy of that area of the body, thus orthogonally suspending said laminar element in relation to the surface of the sanitary napkin. In that position, during the use, said laminar element penetrates, even partially, between the user's labia majora, thus urging any exudate discharge to contact and follow the surface of the laminar element as far as the central area of the sanitary napkin surface. The leakage trend due to discharges of exudates close to the sanitary napkin edges, mainly the most voluminous ones, is thus prevented, since the laminar element conveys the exudate flow to the center of the absorbent pad - the effect is theoretically compared to that attained in a chemistry laboratory, wherein a liquid to be poured inside a beaker flows down a glass rod. Additionally, since the laminar collecting element is sufficiently thin and delicate, the drawback found in prior art products that propitiate the partial insertion of an appendix of the sanitary napkin between the user's labia majora is greatly reduced.

In a preferred way, the laminar collecting element of the sanitary napkin of the invention is equidistantly located with respect to the side edges.

Although not essential, the resiliency of at least the edge of the laminar collecting element (which edge contacts the user's vaginal area) facilitates its orthogonal positioning in relation to the surface of the absorbent element, and consequently its insertion in the user's vaginal area.

As felt with respect to this invention, the laminar collecting element of the sanitary napkin is not aligned with the absorbent element, the result of which is that the movement of the absorbent element, caused by the movement of the user's body, is not integrally or substantially transmitted to the laminar collecting element. That is, the laminar collecting element and the absorbent element have not many parts in common, and those parts that do are minimally joined to one another, in order to prevent the transference of the movement from one to the other - optionally, the laminar collecting element is joined to only two points of the absorbent element, viz, each of the two opposing longitudinal ends which the laminar collecting element is adhered to. In other words, only at one point from each one of the two opposing longitudinal extremities of the absorbent element.

In order to attain an optimized performance of the present invention, it is essential that the laminar collecting element, when the absorbent of the invention is in use, extends from the area in contact with the user's vagina to the surface of the absorbent element, even if it is not joined or otherwise adhered thereto. This is explained by the fact that the vaginal exudate, when contacting any portion of the laminar collecting element, flows along the surface thereof, for example, by capillarity, the purpose of which is to reach the surface of the absorbent, to be absorbed and retained therein. The embodiments that favor a greater contact between the laminar collecting element and the absorbent surface are preferred.

As can be understood from this text, the absorbent element of the sanitary napkin comprises at least a pad of absorbent material, typically a wood pulp fiber block, but it can include other components (such as superabsorbent substrata, other types of fibers and materials, etc.) also in distinct layers (for example, a non-woven layer or perforated plastic film located on the absorbent material itself to contact the user's body, etc.).

The laminar collecting element is comprised of one or more layers of a same material or distinct materials. The preferred material is the non-woven one, without excluding any other, for example, tissue, plastic film, paper, etc. Its thickness, as denoted by the designation "laminar", is greatly lower than the length or width dimensions; i.e its thicknesses is lower than 5 mm, preferably lower than 2 mm. Its surface can be continuous or discontinuous, surfaces having orifices or discontinuities with round, square shaped forms, or of any another format being within the scope of the invention. Also within the scope of the invention, configurations of the laminar collecting element comprise:
- a set of filaments which are spaced apart and/or parallel, with little or no interconnection transverse to the direction of the descending movement of the fluid to be conveyed;
- a corrugated laminar material, of one or more layers associated with one another;
- an arrangement where two or more layers are associated with one another thus forming tubular passages generically parallel to the direction of the descending movement of the fluid to be conveyed.

The laminar conveying element can be hydrophobic, hydrophilic, or concomitantly contain independent portions of opposing hydrophilic character. The affinity for the aqueous medium can be of the material per se or acquired through some treatment or coating.

The laminar collecting element can optionally contain an absorbent material, without departing from the scope of the invention. It can also be provided with lines or embossed areas, that is, more compressed than the material adjacent thereto, in such a way that it affects the speed of liquid distribution.

The laminar collecting element can be configured to be able to penetrate between the user's labia majore, or to simply contact them externally.

### Brief Description of the Drawings

The present invention will be described in full details below based on a practical example represented in the accompanying drawings. The respective figures show:
Figure 1 - a perspective view of a preferred embodiment of the sanitary napkin made in accordance with the present invention; and
Figure 2 - a cut view of the napkin illustrated in Figure 1;

### Detailed Description of the Figures

The sanitary napkin 1 of the present invention is comprised of an elongated absorbent core 6, wrapped by a top layer 3 and a lining layer 4.

The top layer 3 contacts the pelvic area of the user and usually is made of impervious material that does not cause any irritation to the user's skin, for example, non-woven or perforated plastic film.

The purpose of the lining layer 4, opposite the top layer, is to prevent the fluid retained in the absorbent core from passing to the user's clothes and usually is a thin film made of impervious material, for example, polyethylene.

According to the present invention, the napkin 1 is also comprised of a substantially straight and elongated collecting and conveying element 10, substantially disposed along the longitudinal axis of said napkin 1. According to a particular embodiment, said element 10 extends along a longitudinal length corresponding to 2/3 of the length of the napkin 1, but it can extend along said entire length, or extend along shorter lengths, for example less than the half of the length of the napkin 1.

Still according to a preferred embodiment, the conveying element 10 comprises a longitudinally elongated main portion 15 which is preferably elastic (for example, due to the presence of an elastic thread 14), that extends from between the two main attachment points 17 to the element 10 of the napkin 1. A continuous curtain portion 16 vertically extends the main portion 15 as far as the top layer 3 of the napkin 1 along the entire length of the conveying element 10.

Optionally, said curtain portion 16 can be discontinuous, for example, through a plurality of orifices (not shown) perpendicular to the longitudinal axis. Depending on its efficiency, the size of the curtain portion 16 can be reduced.

Said attachment points 17 are preferably provided by joining (that is, any process known to the skilled artisan such as sewing, ultrasonic sealing, adhesivation, and the like) the main portion 15 to the top layer 3 of the napkin 1. Notwithstanding said fact, the ends of the main portion 15 also can be adequately joined to any other elements of the napkin 1, such as the lining layer 4.

The main portion 15 and the eventual curtain portion 16 that are integral with the element 10 may or may not be made of non-absorbent, absorbent, hydrophilic or hydrophobic material. Preferably, said material is a non-woven film and therefor it can be a piece of tissue, a perforated or non-perforated plastic film, laminates or said materials compounded with themselves or other materials, etc. Lower thicknesses of the element 10 are preferred.

The main portion 15 and the curtain portion 16 that are integral with the element 10 of the sanitary napkin 1 according to this invention may optionally be configured as an orthogonal continuation of the top layer 3 of the sanitary napkin 1 itself.

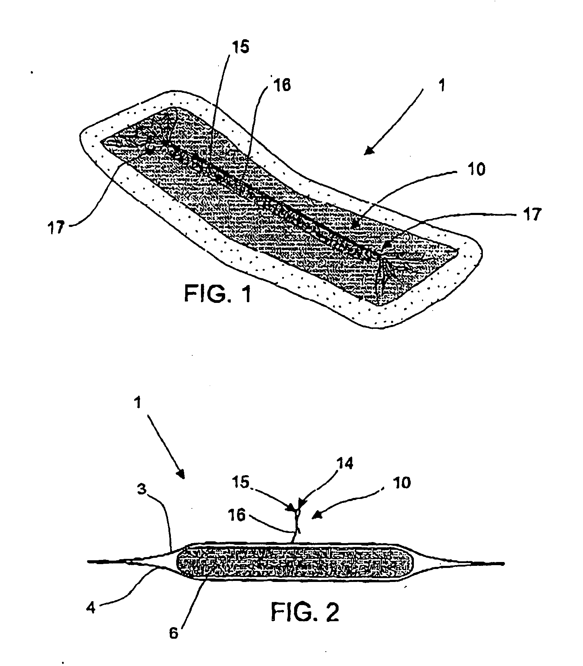

Said main portion 15 also can be made of a non-elastic material. In this case, its length will be preferably shorter than the corresponding longitudinal length of the napkin 1, so that the main portion 15 can be stretched and moved away from the corresponding longitudinal length of the top layer 3 of the napkin 1 when the latter is opened or unfolded, thus facilitating the formation of the straight portion of use illustrated in Figure 1.

Said main portion 15 is preferably conformed as a elongated strap, that represents a prolongation of the curtain portion curtain 16 (alternatively, two or more curtain portions 16 can be present).

In the embodiments illustrated in Figures 1 and 2, the main portion 15, during the use, is partially inserted between the user's labia majora.

In the presence of the curtain portion 16, firstly the vaginal exudate contacts with the main portion 15 of the conveying element 10, and is conveyed later (through the surface tension, capillarity, and the like) by said curtain portion 16 towards the top layer 3 of the napkin 1. Thus, the fluid is advantageously conveyed to a central area of the napkin which is capable to withstand larger fluid discharges than the peripheral areas of the napkin 1 without any leakage. Thus, the possibility of leakage is considerably mitigated.

It is not essential that the curtain portion 16, beyond the attachment points 17, be joined through sealing, gluing or any other method, to the surface of the absorbent element, in this example embodied by the top layer 3 - most importantly, a physical proximity between the curtain portion 16 and the top layer 3 is required so that the fluid can be transferred to and stored in the absorbent core 6.

An optional embodiment (not shown) foresees a main portion 15 including an upward bending in its longitudinal medium portion orthogonal to the surface of the napkin 1, so that it has an increased penetration in the user's vulva area, thus incrementing the advantages of this invention.

After the examples of the preferred embodiments have been described, it should be understood that the scope of the present invention embodies other possible variants, being limited only by the text of the accompanying claims, the possible equivalents being included thereto.

## Claims

1. A sanitary napkin **characterized by** comprising a collecting and conveying element for vaginal exudates and an absorbent element, said collecting and conveying element is substantially laminar in that it comprises one or more layers of the same material or distinct materials and has a thickness lower than 5 mm, and wherein said collecting and conveying element is orthogonal to the surface of said napkin, and not aligned with said absorbent element so that the movement of the absorbent element caused by the movement of the user's body is not integrally or substantially transmitted to the laminar collecting and conveying element, wherein said sanitary napkin comprises a top layer (3), a lining layer (4) and an absorbent core (6), the absorbent core (6) being disposed between the top layer (3) and the lining layer (4), the collecting and conveying element (10) being associated with the top layer (3) or with the lining layer (4) in main attachment points (17) located in extremity and opposing points of the longitudinal axis of the napkin (1), wherein the collecting and conveying element (10) comprises a main portion (15) and a curtain portion (16), the curtain portion (16) extending from the main portion (15) to the top layer (3), and wherein the collecting and conveying element (10) is fully or partially elastic per se or by being associated with an elastic thread (14).

2. The sanitary napkin in accordance with claim 1, **characterized in that** the collecting and conveying element (10) is an orthogonal prolongation of the top layer (3).

3. The sanitary napkin in accordance with claim 1, **characterized in that** the collecting and conveying element is fully or partially made of a non-woven material.

4. The sanitary napkin in accordance with claim 1, **characterized in that** the curtain portion (16) is continuous.

5. The sanitary napkin in accordance with claim 1, **characterized in that** the curtain portion (16) is discontinuous.

6. The sanitary napkin in accordance with claim 1, **characterized in that** the main portion (15) and/or the curtain portion (16) are made of absorbent material.

7. The sanitary napkin in accordance with claim 1, **characterized in that** the main portion (15) and/or the curtain portion (16) are made of a non-absorbent material.

8. The sanitary napkin in accordance with claim 1, **characterized in that** the main portion (15) is configured with an elongated strap that is a prolongation of the curtain portion (16).

9. The sanitary napkin in accordance with claim 1, **characterized in that** said curtain portion (16) of said collecting and conveying element (10) is fully or partially joined to the top layer (3).

10. The sanitary napkin in accordance with claim 1, **characterized in that** said collecting and conveying element has a thickness lower than 2 mm

11. The sanitary napkin in accordance with claim 1, **characterized in that** said collecting and conveying element has a longitudinal length of approximately 2/3 of the longitudinal length of said absorbent element.

## Patentansprüche

1. Damenbinde, **dadurch gekennzeichnet, daß** sie ein Auffang- und Ableitelement für Vaginalexsudate und ein absorbierendes Element umfaßt, wobei das Auffang- und Ableitelement im wesentlichen laminar ist, dergestalt, daß es eine oder mehr Lagen des gleichen Materials oder unterschiedlicher Materialien umfaßt und eine Dicke von weniger als 5 mm aufweist, und wobei das Auffang- und Ableitelement rechtwinklig zur Oberfläche der Binde und nicht in der Weise mit dem absorbierenden Element verbunden ist, daß die Bewegung des absorbierenden Elements, die durch die Bewegung des Körpers der Nutzerin verursacht wird, integral oder substantiell auf das laminare Auffang- und Ableitelement übertragen wird, wobei die Damenbinde eine obere Lage (3), eine Auskleidungslage (4) und einen absorbierenden Kern (6) umfaßt, der absorbierende Kern (6) zwischen der oberen Lage (3) und der Auskleidungslage (4) angeordnet ist, wobei das Auffang- und Ableitelement (10) mit der oberen Lage (3) oder der Auskleidungslage (4) an Hauptbefestigungspunkten (17) befestigt ist, die sich an gegenüberliegenden Endpunkten der longitudinalen Achse der Binde (1) befinden, wobei das Auffang- und Ableitelement (10) einen Hauptabschnitt (15) und einen Vorhangabschnitt (16) umfaßt und der Vorhangabschnitt (16) sich vom Hauptabschnitt (15) zur oberen Lage (3) hin erstreckt und wobei das Auffang- und Ableitelement (10) vollständig oder zum Teil per se oder durch die Verbindung mit einem elastischen Faden (14) elastisch ist.

2. Damenbinde nach Anspruch 1, **dadurch gekennzeichnet, daß** das Auffang- und Ableitelement (10) eine rechtwinklige Verlängerung der oberen Lage (3) ist.

3. Damenbinde nach Anspruch 1, **dadurch gekennzeichnet, daß** das Auffang- und Ableitelement vollständig oder zum Teil aus einem Vliesstoffmaterial hergestellt ist.

4. Damenbinde nach Anspruch 1, **dadurch gekennzeichnet, daß** der Vorhangabschnitt (16) durchgehend ist.

5. Damenbinde nach Anspruch 1, **dadurch gekennzeichnet, daß** der Vorhangabschnitt (16) nicht durchgehend ist.

6. Damenbinde nach Anspruch 1, **dadurch gekennzeichnet, daß** der Hauptabschnitt (15) und/oder der Vorhangabschnitt (16) aus einem absorbierenden Material hergestellt sind.

7. Damenbinde nach Anspruch 1, **dadurch gekennzeichnet, daß** der Hauptabschnitt (15) und/oder der Vorhangabschnitt (16) aus einem nicht absorbierenden Material hergestellt sind.

8. Damenbinde nach Anspruch 1, **dadurch gekennzeichnet, daß** der Hauptabschnitt (15) mit einem länglichen Band gestaltet ist, das eine Verlängerung des Vorhangabschnitts (16) darstellt.

9. Damenbinde nach Anspruch 1, **dadurch gekennzeichnet, daß** der Vorhangabschnitt (16) des Auffang- und Ableitelements (10) vollständig oder zum Teil mit der oberen Lage (3) verbunden ist.

10. Damenbinde nach Anspruch 1, **dadurch gekennzeichnet, daß** das Auffang- und Ableitelement eine Dicke von weniger als 2 mm hat.

11. Damenbinde nach Anspruch 1, **dadurch gekennzeichnet, daß** das Auffang- und Ableitelement eine longitudinale Länge von zirka 2/3 der longitudinalen Länge des absorbierenden Elements aufweist.

## Revendications

1. Serviette hygiénique **caractérisée en ce qu'**elle comprend un élément pour la collecte et le transport des exsudats vaginaux et un élément absorbant, ledit élément pour la collecte et le transport est essentiellement lamellaire du fait qu'il comprend une ou plusieurs couches du même matériau ou de matériaux distincts et possède une épaisseur inférieure à 5 mm, et dans laquelle ledit élément pour la collecte et le transport est perpendiculaire à la surface de ladite serviette, et n'est pas aligné avec ledit élément absorbant de manière à ce que le déplacement de l'élément absorbant provoqué par le déplacement du corps de l'utilisateur n'est pas transmis intégralement ou partiellement à l'élément pour la collecte et le transport, dans laquelle ladite serviette hygiénique comprend une couche supérieure (3), une doublure (4) et un coeur absorbant (6), le coeur absorbant (6) étant disposé entre la couche supérieure (3) et la doublure (4), l'élément pour la collecte et le transport (10) étant associé à la couche supérieure (3) ou à la doublure (4) aux principaux points d'attache (17) situés aux points extrêmes et opposés de l'axe longitudinal de la serviette (1), dans laquelle l'élément pour la collecte et le transport (10) comprend une partie principale (15) et une partie voile (16), la partie voile (16) s'étendant de la partie principale (15) à la couche supérieure (3), et dans laquelle l'élément pour la collecte et le transport (10) est totalement ou partiellement élastique en lui-même ou par association à un fil élastique (14).

2. Serviette hygiénique selon la revendication 1, **caractérisée en ce que** l'élément pour la collecte et le transport (10) est une prolongation perpendiculaire de la couche supérieure (3).

3. Serviette hygiénique selon la revendication 1, **caractérisée en ce que** l'élément pour la collecte et le transport est totalement ou partiellement fait de matériau non-tissé.

4. Serviette hygiénique selon la revendication 1, **caractérisée en ce que** la partie voile (16) est continue.

5. Serviette hygiénique selon la revendication 1, **caractérisée en ce que** la partie voile (16) est discontinue.

6. Serviette hygiénique selon la revendication 1, **caractérisée en ce que** la partie principale (15) et/ou la partie voile (16) sont faites de matériau absorbant.

7. Serviette hygiénique selon la revendication 1, **caractérisée en ce que** la partie principale (15) et/ou la partie voile (16) sont faites de matériau non-absorbant.

8. Serviette hygiénique selon la revendication 1, **caractérisée en ce que** la partie principale (15) est munie d'un ruban allongé qui est une prolongation de la partie voile (16).

9. Serviette hygiénique selon la revendication 1, **caractérisée en ce que** la partie voile (16) dudit élément pour la collecte et le transport (10) est complètement ou partiellement jointe à la couche supérieure (3).

10. Serviette hygiénique selon la revendication 1, **caractérisée en ce que** ledit élément pour la collecte et le transport a une épaisseur inférieure à 2 mm.

11. Serviette hygiénique selon la revendication 1, **caractérisée en ce que** ledit élément pour la collecte et le transport a une longueur longitudinale d'environ 2/3 de la longueur longitudinale dudit élément absorbant.
